**Europäisches Patentamt**

**European Patent Office**

**Office européen des brevets**

(11) Veröffentlichungsnummer : **0 442 140 B1**

## (12) EUROPÄISCHE PATENTSCHRIFT

(45) Veröffentlichungstag der Patentschrift :
**15.09.93 Patentblatt 93/37**

(21) Anmeldenummer : **90125373.2**

(22) Anmeldetag : **22.12.90**

(51) Int. Cl.⁵ : **A23C 9/20, A23C 9/142**

---

(54) **Diätetisches Nahrungsmittel für Patienten mit Niereninsuffizienz.**

(30) Priorität : **26.01.90 DE 4002204**

(43) Veröffentlichungstag der Anmeldung :
**21.08.91 Patentblatt 91/34**

(45) Bekanntmachung des Hinweises auf die
Patenterteilung :
**15.09.93 Patentblatt 93/37**

(84) Benannte Vertragsstaaten :
**AT ES GB IT SE**

(56) Entgegenhaltungen :
**EP-A- 0 326 617**
**PATENT ABSTRACTS OF JAPAN Database**
**JAPS/JPO, Band 14, Nr. 326 (C-740), 12. Juli**
**1990; & JP -A - 2117366 (MORINAGA MILK**
**IND.) 01.05.1990**

(73) Patentinhaber : **MILCHWERKE WESTFALEN**
**eG**
**Bielefelder Strasse 66**
**D-32051 Herford (DE)**

(72) Erfinder : **Behr, Horst, Dr.**
**Im Brakensiek 7**
**W-4900 Herford (DE)**
Erfinder : **Manz, Friedrich, Dr.**
**Bierkamp 17**
**W-4600 Dortmund-Brünninghausen (DE)**

(74) Vertreter : **Stracke, Alexander, Dipl.-Ing. et al**
**Jöllenbecker Strasse 164**
**D-33613 Bielefeld (DE)**

EP 0 442 140 B1

Jouve, 18, rue Saint-Denis, 75001 PARIS

## Beschreibung

Die vorliegende Erfindung betrifft ein diätetisches Nahrungsmittel für Patienten mit Niereninsuffizienz, mit reduzierten Eiweiß- und Phosphoranteilen sowie mit Fetten, Kohlenhydraten und Mineralstoffen.

Bekannte diätetische Nahrungsmittel für Patienten mit Niereninsuffizienz (Grüne Liste 1989, Aulendorf: Edition Cantor, Nummern 12005, 14004) basieren auf einem Eiweißanteil in Form von Oligopeptiden, haben einen Gesamtenergiegehalt von etwa 419 - 544 ka/100 mL (100 - 130 kcal/100 ml) sowie einen Phosphorgehalt von etwa 10 - 13 mg/g Protein. Durch die Bildung des Eiweißanteiles in Form von Oligopeptiden ergeben sich insoweit anwenderseitig erhebliche Probleme, als aufgrund der Strukturzerstörung des Proteins infolge der Oligopeptid-Gewinnung eine küchentechnische Verarbeitbarkeit nicht mehr sinnvoll gegeben ist, die sensorischen Eigenschaften sich beispielsweise von denen einer Kuhmilch erheblich unterscheiden und insbesondere auch der sehr schlechte Geschmack von Oligopeptiden die Akzeptanz beim Patienten außerordentlich negativ beeinträchtigt, vor allen Dingen wenn eine Daueranwendung erforderlich ist. Der Gesamtenergiegehalt erscheint relativ gering. Der Phosphatgehalt liegt an der oberen Grenze des Anzustrebenden.

Aus der WO 88/02219 ist eine phosphorreduzierte Milch bekannt, die mit der dort angegebenen Untergrenze des Phosphatgehaltes in den Bereich der Obergrenze eines anzustrebenden Phosphatgehaltes liegt. Der Eiweißanteil basiert auf Casein-Proteinen. Diese werden durch Ausfällung mit Milchsäure aus einer Magermilch gewonnen. Aus der verbleibenden Molke wird Phosphorsäure mittels Calcium ausgefällt. Danach wird das Casein wieder zugefügt. Da infolge der Ausfällung des Caseins und seiner erneuten Zufügung das im Augenblick der Ausfällung noch in den Caseinproteinen eingeschlossene Phosphor in jedem Fall im Produkt verbleibt, lassen sich durch die Verwendung dieser Caseinproteine anzustrebende niedrige Phosphorwerte nicht erreichen. Die Milchsäurebehandlung kann zumindest tendentiell zu Strukturveränderungen führen, die die küchentechnische Verarbeitung erschweren können. Der weitaus überwiegende Anteil des Proteins stammt aus dem Casein und hat von daher in nachteiliger Weise einen relativ geringen Anteil an essentiellen Aminosäuren, die andererseits eine sehr hohe Wertigkeit für den Patienten hätte. Im Hinblick auf den Gewinnungsvorgang der Caseinproteine durch Ausfällung und deren Rekonstitution im Produkt kann eine derartige Nahrung nur in flüssiger Form in den Handel gebracht werden.

Aus der Grünen Liste 1989, Aulendorf, Edition Cantor, Nr. 01005 ist ferner ein eiweißarmes Getränk unter anderem aus Molkebestandteilen bekannt, das indikationsmäßig auf Aminosäurenstoffwechselstörungen ausgelegt ist, die beim Patienten zur Bildung giftiger Stoffwechselprodukte führen. Das Produkt hat einen erhöhten Phosphorgehalt von mehr als 38 mg/g Protein, der eine Verabreichung an Patienten mit Niereninsuffizienz verbietet. Der Gesamtenergiegehalt ist für Patienten mit Niereninsuffizienz deutlich zu niedrig.

Der vorliegenden Erfindung liegt die Aufgabe zugrunde, ein diätetisches Nahrungsmittel für Patienten mit Niereninsuffizienz zu schaffen, das, auch bei Daueranwendung, bei denkbar niedrigem Phosphatgehalt die kalorisch ausreichende Versorgung des Patienten sowie die Versorgung des Patienten mit einem Eiweiß mit hohem Anteil an essentiellen Aminosäuren, unter Beachtung des Erfordernisses der Eiweißreduktion als solcher, gewährleistet, und zwar in Verbindung mit einem schmackhaften, küchentechnisch gut verarbeitbaren Produkt.

Die erfindungsgemäße Lösung ergibt sich aus dem kennzeichnenden Teil des Patentanspruches 1. Die hohe Akzeptanz dieses diätetischen Nahrungsmittels beim Patienten resultiert daraus, daß es sensorisch und küchentechnisch milchähnlich ist, also im Geschmack, im Geruch und im Aussehen milchähnlich ist und auch wie Milch verarbeitet werden kann, also beispielsweise warm oder kalt getrunken, aber auch zu weiteren zahlreichen und dauerhaft schmackhaften Rezepturen insbesondere auf lactovegetabiler Basis verarbeitet werden kann. Trotz der Beachtung der diätetischen Grundprinzipien einer Diätbehandlung bei Niereninsuffizienz kann dieses Nahrungsmittel mit seinem hohen Energiegehalt als ausgesprochen kalorienreich bezeichnet werden, wie der Energiegehalt von 209 - 2093 ka/100 ml (50 - 500 kcal/100 ml) aufzeigt. Im Vergleich hierzu ist darauf zu verweisen, daß Kuhmilch im allgemeinen einen Energiegehalt in der Größenordnung von 276 ka/100 ml (66 kcal/100 ml) hat und bereits als energiereiches Getränk bezeichnet wird. Wichtig ist aber dabei, daß das erfindungsgemäße diätetische Nahrungsmittel im Sinne der Diätvorschrift eiweißarm ist, da nur 1 - 10 % des genannten gesamten Energiegehaltes vom Proteinanteil des Nahrungsmittels gebildet werden, wobei im Hinblick auf die Diätvorschriften sehr wesentlich ist, daß der Phosphorgehalt unter 10 mg/g des Proteins eingestellt ist. Trotz der Milchähnlichkeit ist das Nahrungsmittel ausgesprochen phosphorarm. Hervorzuheben ist ferner, daß der Proteinanteil im wesentlichen aus Molkeprotein gebildet ist, wobei in diesem Zusammenhang das Wort "Protein" in seinem eigentlichen Sinn, nämlich als natives Protein zu verstehen ist. Diese Erscheinungsform trägt wesentlich mit dazu bei, das Produkt küchentechnisch und sensorisch der Kuhmilch nachzubilden. Die Verwendung von Molkeproteinen hat den großen Vorteil, daß hierdurch dem Patienten ein optimaler Anteil an essentiellen Aminosäuren angeboten wird, der trotz des Erfordernisses der Eiweißarmut nunmehr noch in Höhe des Tagesbedarfes des Patienten gehalten werden kann, ohne die Harnstoffmenge in einen kritischen Bereich zu erhöhen.

Weitere zweckmäßige Ausgestaltungen sind in den Unteransprüchen gekennzeichnet. Hervorzuheben ist insoweit ein vorgesehener Calciumbereich von bis zu 300 mg/100 ml. Das Produkt ist somit calciumreich und ähnelt auch von daher Milch und Milchprodukten. Die bei den üblichen Niereninsuffizienz-Diäten erforderliche zusätzliche dauerhafte und auf Dauer geschmacklich sehr störende Behandlung mit Calciumtabletten kann bei dieser Ausstattung des Produktes zum Teil entfallen. Die hohe Dosis Calcium hat für Patienten mit Niereninsuffizienz darüber hinaus den Vorteil, daß die Calciumaufnahmemenge aus dem Darm weitgehend normalisiert und die Phosphoraufnahme aus dem Darm gleichzeitig durch die Bildung von unlöslichem Calciumphosphat im Darm vermindert wird.

Der Fettgehalt des Nahrungsmittels wird vorzugsweise aus ernährungsphysiologischer Sicht aus pflanzlichen Fetten gebildet.

Die Erfindung wird nachstehend, auch unter Einschluß eines speziellen Durchführungsbeispieles, näher beschrieben.

Nach seiner grundsätzlichen Zusammensetzung besteht das diätetische Nahrungsmittel gemäß der Erfindung aus Proteinen, Fetten, Kohlenhydraten und Mineralstoffen in einer noch näher zu erläuternden Detailzusammensetzung. Es ist aus diesen Stoffen sensorisch und küchentechnisch kuhmilchähnlich zusammengesetzt, hat also einen kuhmilchähnlichen Geschmack und Geruch, ein kuhmilchähnliches Aussehen und verhält sich physikalisch und chemisch in der Küchentechnologie wie Kuhmilch, kann also in der gleichen Weise entweder kalt oder warm getrunken oder in diversen geschmacklich ansprechenden Rezepturen verarbeitet werden.

Die genannten Bestandteile werden in eine Zusammensetzung gerbacht derart, daß der Energiegehalt des Nahrungsmittels 209 - 2093 ka/100 ml (50 - 500 kcal/100 ml) beträgt. Der Gesamtenergiegehalt ist also hoch. Das Nahrungsmittel ist kalorienreich und verhindert damit die bei vielen Patienten bei derartigen Diäten beobachtete kalorische Unterversorgung. Im Vergleich hierzu sei darauf hingewiesen, daß Kuhmilch selbst einen Energiegehalt von 276 ka/100 ml (66 kcal/100 ml) hat und bereits als energiereiches Getränk bezeichnet wird. Obstsäfte, Gemüsesäfte und dergleichen weisen beispielsweise einen Energiegehalt von unter 209 ka/100 ml (50 kcal/100 ml) auf, Für das erfindungsgemäße Nahrungsmittel hat sich ein Energiegehalt von 419 - 837 ka/100 ml (100 - 200 kcal/100 ml) als besonders zweckmäßig erwiesen.

Wesentlich ist nun im Hinblick auf die diätetischen Vorgaben bei Niereninsuffizienz, daß an dem genannten Gesamtenergiegehalt der Energiegehalt des Proteinanteiles am Nahrungsmittel nur 1 - 10 % beträgt. Die höheren Prozentbereiche sind dabei für Dialysepatienten sinnvoll, bei denen infolge der Verluste von Proteinen und Eiweißbausteinen bei der Dialyse der Eiweißgehalt im Nahrungsmittel etwas höher sein darf als bei Patienten, die keiner Dialyse bedürfen. Ein besonders zweckmäßiger Prozentbereich liegt zwischen 3 und 7 %. Im unteren Bereich ist das Nahrungsmittel immer noch als extrem eiweißarm zu bezeichnen. Auch im oberen Bereich ist das Nahrungsmittel immer noch als durchaus eiweißarm zu bezeichnen, berücksichtigt aber besonders die bei einzelnen Gruppen von Patienten zulässige höhere Eiweißzufuhr.

Wesentlich ist in diesem Zusammenhang, daß möglichst dafür Sorge getragen wird, den ja insgesamt relativ geringen Eiweißanteil möglichst in Form biologisch besonders hochwertigen Eiweißes zuzuführen. Von daher hat es sich als besonders zweckmäßig erwiesen, den Proteinanteil im wesentlichen aus Milcheiweiß zu bilden. Hierbei hat es sich wiederum als besonders zweckmäßig erwiesen, den Proteinanteil im wesentlichen aus Molke zu gewinnen. Dies kann beispielsweise dadurch geschehen, daß Molke, deren Proteingehalt in der Regel 0,6 - 0,8 % beträgt, ultrafiltriert wird. Dadurch werden die Inhaltsstoffe der Molke nach ihrem Molekulargewicht getrennt. Niedermolekulare Stoffe wie Wasser, Lactose und Salze können die Membranen passieren, während die hochmolekularen Molkenproteine zurückgehalten werden. Es lassen sich unterschiedliche Konzentrationsgrade einstellen. Der Eiweißgehalt des für das Nahrungsmittel gemäß der Erfindung verwendeten Molkeproteinprodukts liegt zwischen 40 und 80 %. Wesentlich ist dabei, daß bei diesen Molkeproteinen der Anteil an essentiellen Aminosäuren, also an denjenigen Eiweißbausteinen, die der menschliche Körper selbst nicht bilden kann, besonders hoch ist.

Im Mineralstoffbereich des Nahrungsmittels wird insbesondere bei der Zusammensetzung dafür Sorge getragen, daß der Phosphorgehalt gering ist. Er liegt unter 10 mg/g Protein, in einem typischen Anwendungsfall bei 5 mg/g Protein. Eine niedrige Phosphorzufuhr ist ein wesentliches Ziel für die diätetische Dauerbehandlung von Patienten mit Niereninsuffizienz.

In weiterer zweckmäßiger Ausgestaltung wird im Mineralstoffbereich dafür Sorge getragen, daß ein hoher Calciumgehalt gegeben ist. Das kann bis zu 300 mg/100 ml gehen und sollte eine Untergrenze von 25 mg/100 ml nicht unterschreiten. Der Calciumgehalt der Milch beträgt beispielsweise 120 mg/100 ml. In einer bevorzugten Zusammensetzung ist der Calciumgehalt des Nahrungsmittels dem der Milch naheliegend eingestellt. Milch und Milchprodukte stellen die wichtigste Calciumquelle in der Ernährung dar. Sie werden allgemein als die calciumreichen Lebensmittel schlechthin betrachtet. Ein hoher Calciumgehalt rechtfertigt die ernährungsphysiologischen Erwartungen von Konsumenten allgemein und nierenkranken Patienten im Besonderen an ein Milchprodukt. Darüber hinaus sind Patienten mit Niereninsuffizienz infolge einer krankheitsspezifischen Verminderung der Calciumaufnahme aus dem Darm auf eine hohe Calciumzufuhr angewiesen und vermindert eine hohe Calciumzufuhr die Phosphoraufnahme aus dem Darm in erwünschter Weise durch die Bildung von unlöslichem Calciumphosphat im Darm.

Im Hinblick auf den niedrigen Proteingehalt muß der relativ hohe Gesamtenergiegehalt des Nahrungsmittels über die Komponenten Fett und Kohlenhydrate eingestellt werden. Bei grundsätzlicher Eignung auch tierischer Fette ist aus ernährungsphysiologischer Sicht die bevorzugte Verwendung pflanzlicher Fette besonders zweckmäßig. Bei den Kohlenhydraten wird aus ernährungsphysiologischer Sicht der Schwerpunkt zweckmäßig von Dextrinen gestellt.

Das diätetische Nahrungsmittel gemäß der Erfindung wird zweckmäßig in Bezug auf die üblichen Herstellungstechniken seiner Bestandteile in Pulverform in den Verkehr gebracht.

Die fertige Zubereitung, die milchähnlich sein soll, erreicht man durch Zugabe von abgekochtem Wasser und Verrühren des Pulvers im Wasser.

Als typisches Beispiel für ein diätetisches Nahrungsmittel gemäß der Erfindung ist auf die nachfolgende Analysentabelle zu verweisen, die im Detail die Zusammensetzung eines derartigen Ausführungsbeispieles aufzeigt. In der ersten Spalte sind die Bestandteile je 100 g Pulverform dargestellt, in der zweiten Spalte die Zusammensetzung je 100 Milliliter des diätetischen Nahrungsmittels. In Spalte 3 ist im Vergleich dazu die Zusammensetzung von 100 Milliliter Trinkmilch aufgeführt.

| TABELLE: | | 100g Pulver des diät. Nahrungsmittels | 100 ml des diät. Nahrungsmittels | 100 ml Trinkmilch |
|---|---|---|---|---|
| Protein | g | 4,7 | 1,0 | 3,3 |
| Alanin | mg | | 52 | 120 |
| Arginin | mg | | 28 | 120 |
| Asparaginsäure | mg | | 101 | 280 |
| Cystin | mg | | 17 | 26 |
| Glutaminsäure | mg | | 178 | 750 |
| Glycin | mg | | 20 | 80 |
| Histidin | mg | | 19 | 89 |
| Isoleucin | mg | | 66 | 210 |
| Leucin | mg | | 107 | 350 |
| Lysin | mg | | 90 | 260 |
| Methionin | mg | | 22 | 84 |
| Phenylalanin | mg | | 34 | 170 |
| Prolin | mg | | 52 | 350 |
| Serin | mg | | 48 | 190 |
| Threonin | mg | | 70 | 150 |
| Tryptophan | mg | | 15 | 46 |
| Tyrosin | mg | | 30 | 170 |
| Valin | mg | | 63 | 230 |
| Fett | g | 18,7 | 4,0 | 3,5 |
| Kohlenhydrate | g | 70,2 | 15,0 | 4,6 |
| Glucose | g | 0,4 | 0,1 | |
| Lactose | g | 4,7 | 1,0 | 4,6 |
| Maltose | g | 2,3 | 0,5 | |
| Dextrine | g | 62,9 | 13,4 | |
| Mineralstoffe | mg | 1100 | 230 | |
| Natrium | mg | 96 | 20 | 50 |
| Kalium | mg | 30 | 6 | 155 |
| Chlor | mg | 20 | 4 | 100 |
| Calcium | mg | 565 | 120 | 120 |
| Magnesium | mg | 9 | 2 | 12 |
| Phosphor | mg | 23 | 5 | 90 |
| Energie | kJ | 1984 | 423 | 279 |
| | kcal | 468 | 100 | 67 |
| Osmolarität | mosmol/l | | 170 | 250 |

Die Analysentabelle zeigt bei den Proteinen den hohen Anteil sogenannter essentieller Aminosäuren, also biologisch besonders wertvoller Eiweißbausteine auf, wozu auf die Anteile der in dem Protein enthaltenen Aminosäuren Isoleucin, Leucin, Lysin, Methionin, Phenylalanin, Threonin, Tryptophan und Valin zu verweisen ist.

Die in der Tabelle angegebene Osmolarität charakterisiert die Anzahl der gelösten Stoffteilchen in der Flüssigkeit.

**Patentansprüche**

1. Diätetisches Nahrungsmittel für Patienten mit Niereninsuffizienz, mit reduzierten Eiweiß- und Phosphatanteilen, sowie mit Fetten, Kohlenhydraten und Mineralstoffen, **dadurch gekennzeichnet**, daß der Eiweißanteil aus nativem, im wesentlichen aus Molke gewonnenen Protein gebildet ist, das Nahrungsmittel aus dem nativen Molkeprotein, den Fetten, Kohlenhydraten und Mineralstoffen sensorisch und küchentechnisch kuhmilchähnlich zusammengesetzt ist, einen Gesamtenergiegehalt von 209 - 2093 ka/100 ml (50 - 500 kcal/100 ml) aufweist, 1 - 10 % dieses Gesamtenergiegehaltes durch den Proteinanteil gebildet ist und der Phosphorgehalt unter 10 mg/g Protein beträgt.

2. Diätetisches Nahrungsmittel nach Anspruch 1, dadurch gekennzeichnet, daß es einen Calciumgehalt von bis zu 300 mg/100 ml aufweist.

3. Diätetisches Nahrungsmittel nach Anspruch 1, dadurch gekennzeichnet, daß der Gesamtenergiegehalt des Nahrungsmittels 419 - 837 ka/100 ml (100 - 200 kcal/100 ml) beträgt.

4. Diätetisches Nahrungsmittel nach einem der Ansprüche 1 bis 3, dadurch gekennzeichnet, daß 3 - 7 % des Gesamtenergiegehaltes von dem Proteinanteil gebildet sind.

5. Diätetisches Nahrungsmittel nach Anspruch 1, dadurch gekennzeichnet, daß der Fettgehalt im wesentlichen aus pflanzlichen Fetten gebildet ist.

6. Diätetisches Nahrungsmittel nach Anspruch 1, dadurch gekennzeichnet, daß als Kohlenhydrate im wesentlichen Dextrine vorgesehen sind.

**Claims**

1. A dietetic food for patients with renal insufficiency, with reduced albumin and phosphate contents, and with fats, carbohydrates and mineral substances, characterised in that the albumin content is formed from native protein obtained essentially from whey, the food consisting of the native whey protein, the fats, carbohydrates and mineral substances is of a cow's milk-like composition from the sensory and cooking point of view, it has a total energy content of 209 - 2093 kJ/100ml (50 - 500 kcal/100 ml), 1 - 10% of that total energy content is formed by the protein content, and the phosphorus content is below 10 mg/g of protein.

2. A dietectic food according to claim 1 characterised in that it has a calcium content of up to 300 mg/100 ml.

3. A dietetic food according to claim 1 characterised in that the total energy content of the food is 419 - 837 kJ/100 ml (100 - 200 kcal/100 ml).

4. A dietectic food according to one of claims 1 to 3 characterised in that 3 - 7% of the total energy content is formed by the protein content.

5. A dietetic food according to claim 1 characterised in that the fat content is essentially formed from vegetable fats.

6. A dietetic food according to claim 1 characterised in that dextrins are essentially provided as the carbohydrates.

**Revendications**

1. Aliment diététique destiné à des patients présentant une insuffisance rénale, comprenant une quantité réduite de composants protéiniques et phosphatés et comprenant des lipides, des glucides et des substances minérales, caractérisé en ce que les composants protéiniques sont constitués de protéines natives extraites essentiellement du petit-lait, en ce que l'aliment constitué de protéines natives du petit-lait, de lipides, de glucides et de substances minérales est composé, d'un point de vue sensoriel et culinaire, comme du lait de vache, en ce que la valeur énergétique globale est de 209-2093 kJ/100 ml (50 - 500 kcal/100 ml), en ce que 1-10% de cette valeur énergétique globale est constitué par des composants protéiniques et en ce que la teneur en phosphate représente moins de 10 mg/g de protéine.

2. Aliment diététique selon la revendication 1, caractérisé en ce que celui-ci contient jusqu'à 300 mg/100 ml de calcium.

3. Aliment diététique selon la revendication 1, caractérisé en ce que la valeur énergétique globale de l'aliment est de 419-837 kJ/100 ml (100-200 kcal/100 ml).

4. Aliment diététique selon l'une des revendications 1 à 3, caractérisé en ce que 3-7 % de la valeur énergétique globale est constituée par le composant protéinique.

5. Aliment diététique selon la revendication 1, caractérisé en ce que les lipides présents dans l'aliment sont constitués essentiellement de graisses végétales.

6. Aliment diététique selon la revendication 1, caractérisé en ce qu'est prévue essentiellement, comme glucide, de la dextrine.